# EUROPEAN PATENT APPLICATION

(11) **EP 4 644 412 A1**
(43) Date of publication of application: **05.11.2025**
(21) Application number: 23912468.8
(22) Date of filing: 21.09.2023
(51) Int. Cl.: C07K 14/78, C12N 15/85, C07K 1/22, C12N 15/62

(54) **MANUFACTURE OF ELASTIN-LIKE POLYPEPTIDE-BASED RECOMBINANT PROTEIN AND ANIMAL CELL EXPRESSION PLATFORM THEREOF**

(30) Priority: 27.12.2022 KR 20220186320; 13.09.2023 KR 20230121573
(71) Applicant: NexThera Co., Ltd., Busan 48931 (KR)
(72) Inventor: JUNG, Ha Na, Busan 47328 (KR); ROH, Kug Hwan, Busan 49418 (KR); LIM, Na Young, Busan 47387 (KR); KIM, Min Jung, Busan 49253 (KR)
(74) Representative: Mewburn Ellis LLP
(86) International application number: PCT/KR2023/014433
(87) International publication number: WO 2024/143785

(57) **Abstract**

The present invention relates to the manufacture of an elastin-like polypeptide-based recombinant protein and an animal cell expression platform thereof, particularly, an animal cell expression platform using a fusion recombinant protein comprising: a repeating elastin-based polypeptide (EBPn, wherein n represents the number of repeating basic unit of EBP); and an antibody constant region (Fc) connected to the EBPn, and the technology of EBPn-Fc recombinant protein animal cell expression platform that can fuse various proteins including peptides, proteins, or antibody fragments and the like, in the front and back of the EBPn-Fc or on both sides of the front and back of the EBPn-Fc. In the EBPn-Fc animal cell expression platform of the present invention, proteins, such as peptides, proteins, antibody fragments, and the like, are expressed in a fused form with the EBPn-Fc, enabling high-purity purification and forming a nanostructure while the functionality of the fusion proteins is maintained. Thus, the EBPn-Fc animal cell expression platform can be used for research and therapeutic applications of recombinant proteins.

## Description

### Technical Field

The present disclosure relates to development and utilization of a recombinant protein based on a fusion recombinant protein including a repetitive hydrophilic elastin-like polypeptide (EBPn); and an antibody constant region (Fc) linked to the EBPn, and an animal cell expression platform.

### Background Art

Elastin is a major component of extracellular matrix (ECM) proteins, composed of an elastomeric domain and a crosslinking domain. The elastomeric domain consists of hydrophobic amino acids and repetitive peptides such as VPGG, VPGVG, and APGVGV, based on which stimuli-responsive, biocompatible, biodegradable, and non-immunogenic elastin-based polypeptides (EBPs) have been designed. The elastin-based polypeptides (EBPs) having pentapeptide repeating units are thermoresponsive biopolymers, and many studies have been conducted since Val-Pro-(Gly or Ala)-Xaa-Gly (where the Xaa is any amino acid except Pro) is able to modulate the responsiveness to environmental changes.

The EBPs undergo a reversible phase transition at a lower critical solution temperature (LCST), which is called transition temperature (Tt). They are in a soluble state below Tt but become insoluble as the temperature increases above Tt. These thermoreversible phase transitions enable utilization of simple purification methods such as inverse transition cycling (ITC) and self-assembly into particles, gels, fibers, and other structures by being triggered by heat. However, in order to purify to a purity suitable for a therapeutic use, ITC purification requires multiple purification processes, and processes such as endotoxin elimination may be involved depending on the protein expression system.

In general, the physicochemical properties of EBPs are mainly regulated by combination of pentapeptide repeating units, Val-Pro-(Gly or Ala)-Xaa-Gly. Specifically, the third amino acid within the repeating unit shows mechanical properties, e.g., elasticity by Gly and plasticity by Ala, whereas the fourth amino acid Xaa and polymerization of the pentapeptide repeating unit affect Tt. Depending on the combination of pentapeptide repeating units, EBPs with various physicochemical properties and Tt may be prepared.

EBPs may be fused to other functional peptides or proteins to provide functional multivalency. In the case of peptides, they have low toxicity as signaling molecules, low incidence of side effects, and highly selective inducibility for intracellular effects, but their short half-life limits in-vivo applicability. Cytokines also have high functional utility, but there are limits in utilization due to their short half-life in vivo. For an increase in the half-life of peptides and recombinant proteins having short half-life, PEGylation to modify proteins with poly(ethylene glycol) (PEG) is being applied. However, since PEGylation is a chemical modification after the production of recombinant proteins, multiple purification processes are required, which increases the production cost, changes in elongation shape in the long-term use, and causes immune responses to PEG.

EBPs are used as inactive protein-based biomaterials such as PEG, are applied in advanced drug delivery systems, regenerative medicine, and tissue engineering, and also function as drug delivery carriers along with drugs or other functional proteins.

EBPs have the advantage of being able to be purified by centrifugation without chromatography based on their property of being reversibly aggregated by heat stimulation and are utilized as biomaterials owing to their low immunogenicity, and the size control and nanocomplex formation are allowed through genetic recombination, such that there is also a applicability for an increase in the half-life of protein therapeutics in vivo and as a drug delivery platform. However, there are limitations, such as formation of intracellular inclusion bodies when they are expressed in E. coli or a poor capability to provide sufficient purification efficiency when they are expressed in animal cells.

### Disclosure of the Invention

### Technical Goals

An object of the present disclosure is to provide an animal cell expression cassette including a gene encoding a hydrophilic elastin-like polypeptide (EBPn) and a gene encoding a protein for expression linked to the front and/or back of a gene encoding an antibody constant region (Fc), a recombinant expression vector including the animal cell expression cassette, and a cell line transfected with the recombinant expression vector.

In addition, another object of the present disclosure is to provide a method of producing a protein for expression, including culturing the cell line and recovering a culture solution; and isolating and purifying a protein for expression fused with EBPn and Fc from the recovered culture solution.

### Technical Solutions

To achieve the above object, the present disclosure provides an animal cell expression cassette including a gene encoding a hydrophilic elastin-like polypeptide (EBPn), a gene encoding an antibody constant region (Fc), and a gene encoding a first protein for expression, and an animal cell expression cassette further including a gene encoding a second protein for expression.

In addition, the present disclosure provides a recombinant expression vector including the animal cell expression cassette and a cell line transfected with the recombinant expression vector.

In addition, the present disclosure provides a method of producing a protein for expression, including culturing the cell line and recovering a culture solution, and isolating and purifying a protein for expression fused with EBPn and Fc from the recovered culture solution.

### Advantageous Effects

The present disclosure relates to production of a recombinant protein based on an elastin-like polypeptide and an animal cell expression platform thereof, and more particularly, as an animal cell expression platform using a fusion recombinant protein including a repetitive elastin-like polypeptide (EBPn, n is the number of repetitions of an EBP basic unit); and an antibody constant region (Fc) linked to the EBPn, the present disclosure relates to an animal cell expression platform technology for an EBPn-Fc recombinant protein, capable of fusing various proteins, such as peptides, proteins, and antibody fragments, to the front and back or both sides of EBPn-Fc. The EBPn-Fc animal cell expression platform of the present disclosure expresses proteins such as peptides, proteins, and antibody fragments in a form fused with EBPn-Fc, allows high-purity purification, and forms nanostructures while maintaining a functionality of the fusion protein, thereby enabling application of recombinant proteins to research and therapeutic fields.

### Brief Description of Drawings

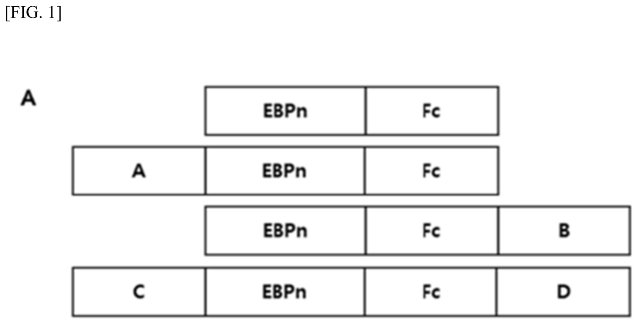

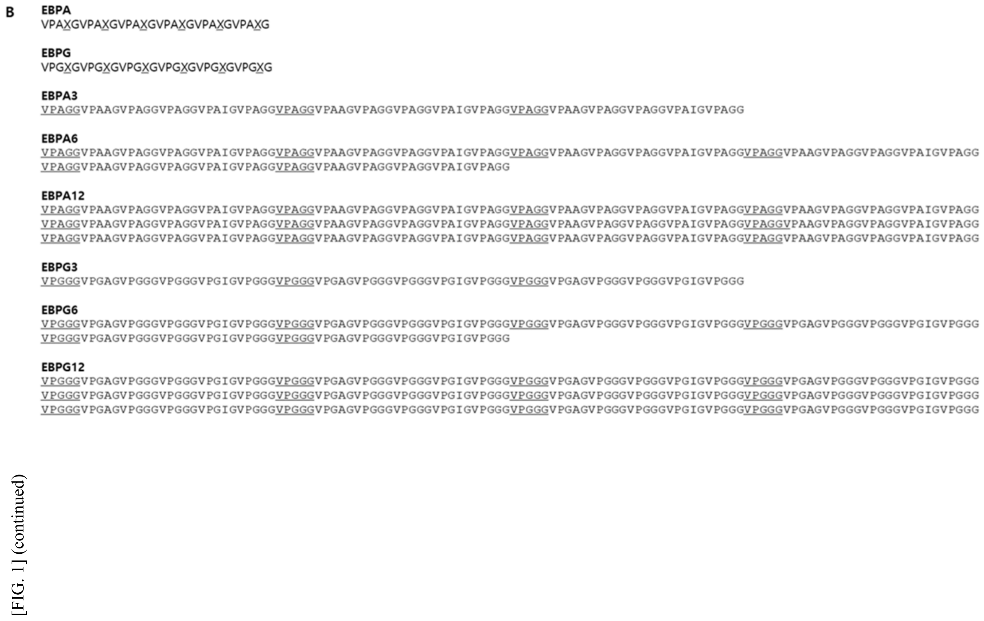

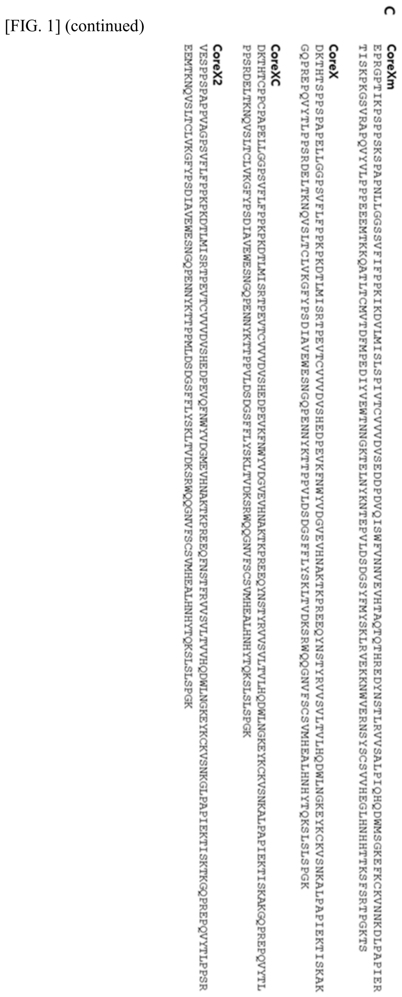

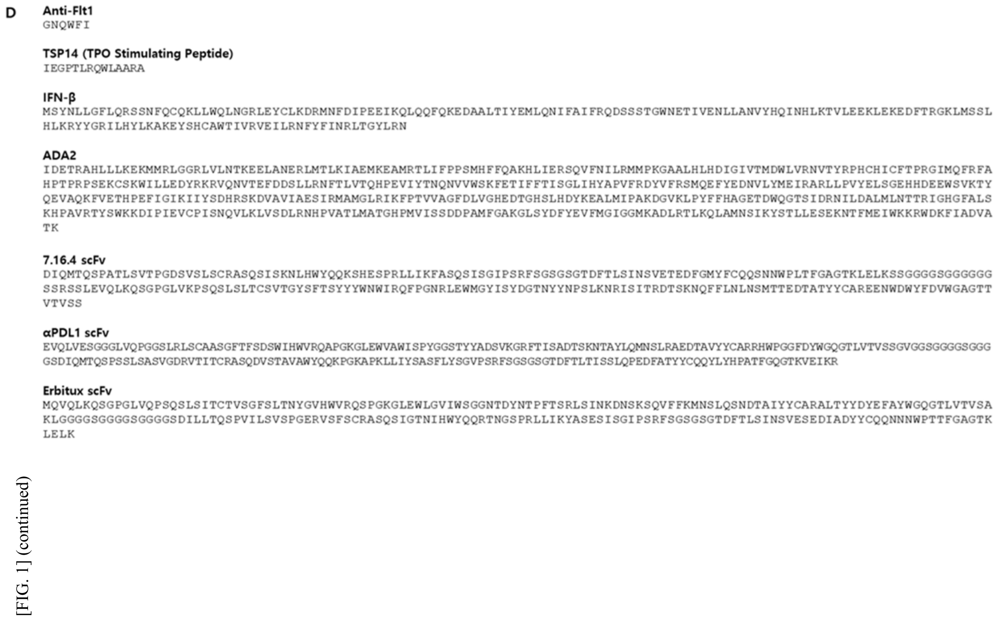

FIG. 1 shows structures and amino acid sequences of an EBPn-Fc fusion protein, wherein FIG. 1A shows an outline of structures of an EBPn-Fc fusion recombinant protein using a pSecTag2 vector, FIGS. 1B and 1C show amino acid sequences of EBPn-Fc, which is a basic structure produced for protein fusion, and FIG. 1D shows amino acid sequences of a peptide, protein, and antibody fragment fused with EBPn-Fc.

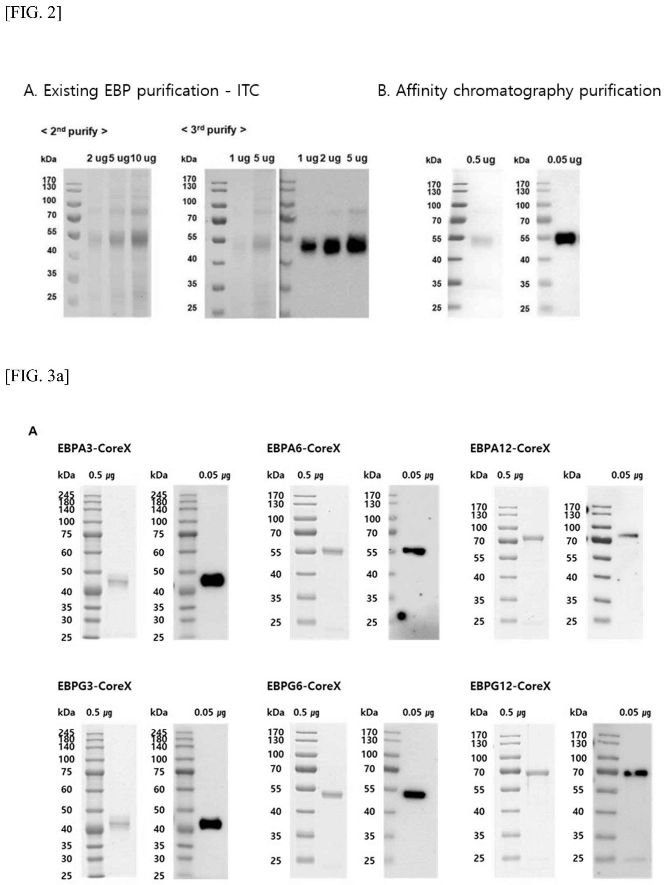

FIG. 2 shows results of comparing purity by purifying each EBPn-Fc fusion protein produced using animal cells via ITC purification, which is a conventional EBP purification method, and affinity chromatography purification using a protein A column.

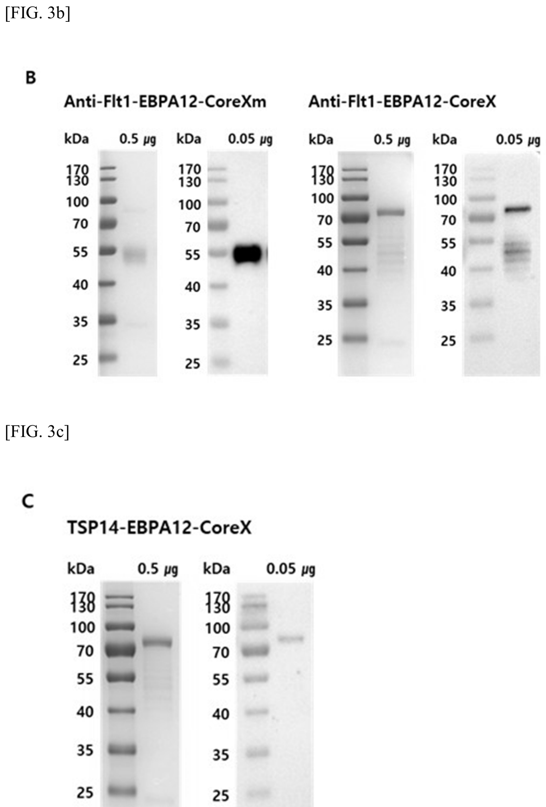

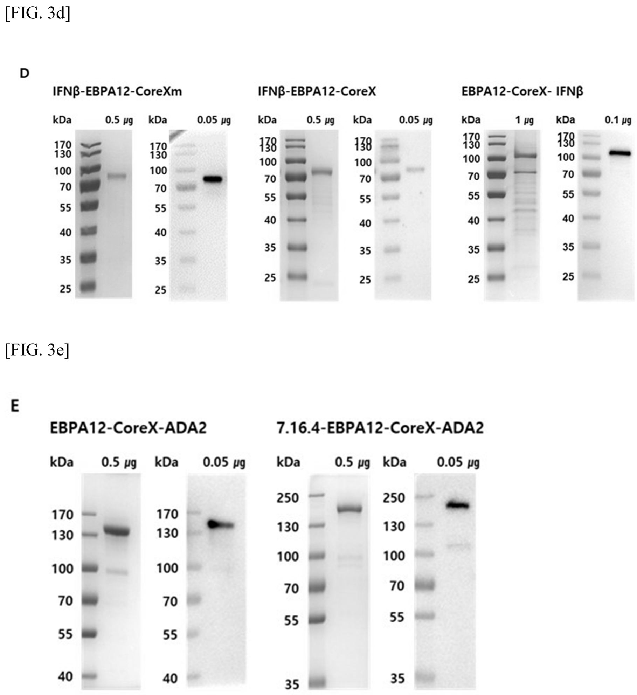

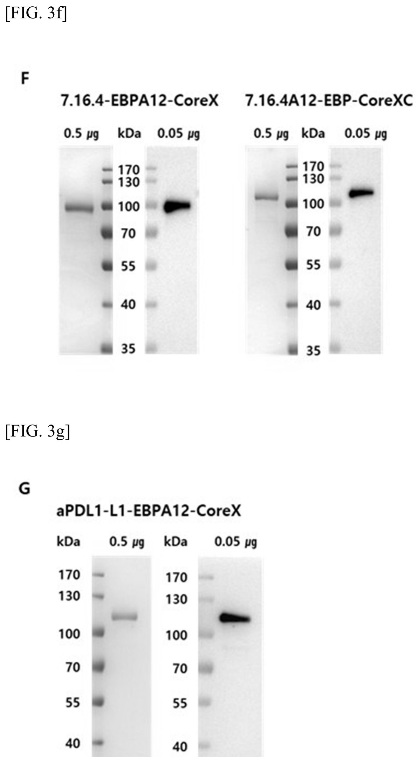

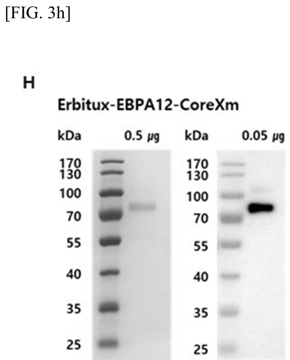

FIG. 3 shows results of identifying a size and purity by purifying each fusion protein produced using animal cells, illustrating results obtained by performing SDS-PAGE and Western blot after purification of a basic EBPn-Fc protein in FIG. 3A, a peptide fusion protein in FIGS. 3B and 3C, a cytokine in FIG. 3D, an enzyme fusion protein in FIG. 3E, and an antibody fragment fusion protein in FIGS. 3F, 3G, and 3H.

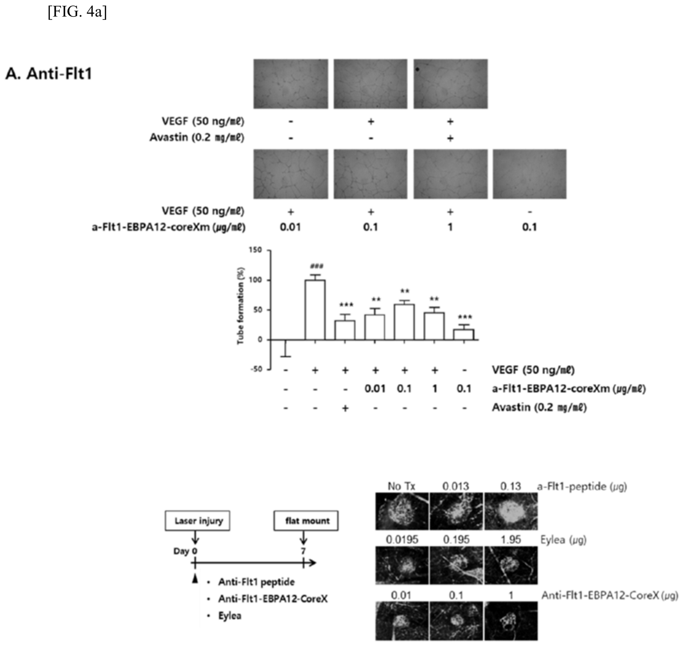

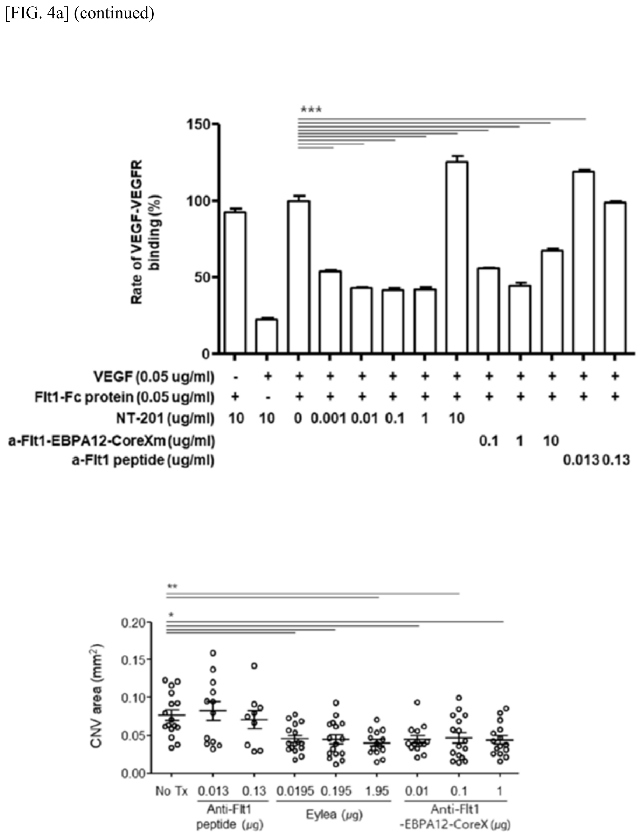

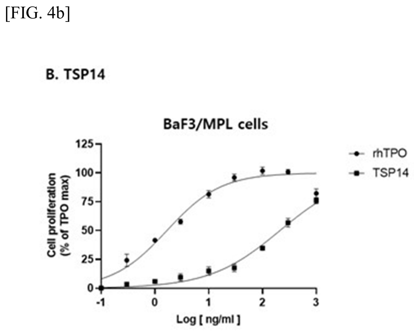

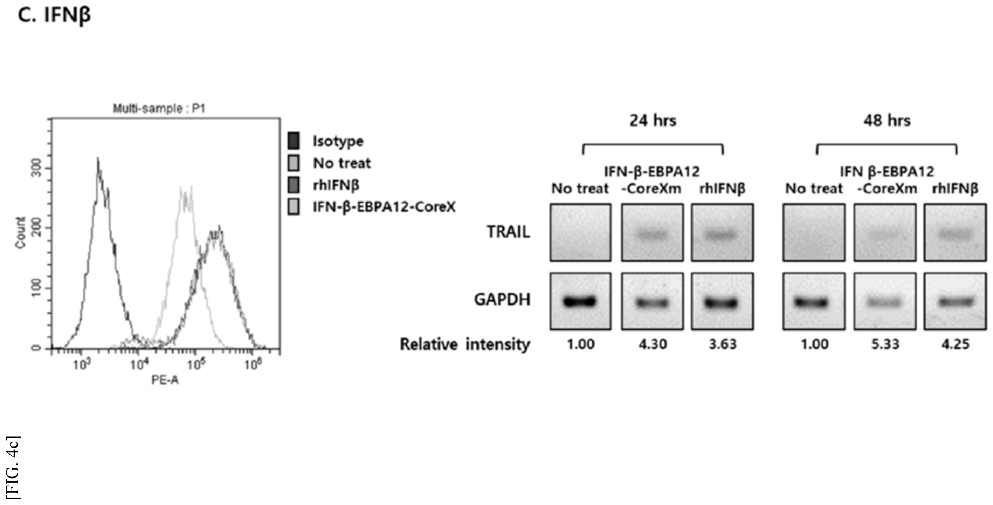

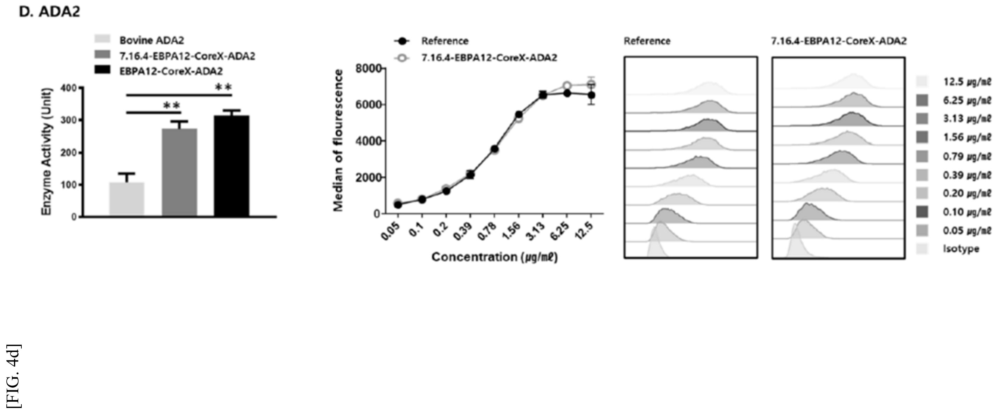

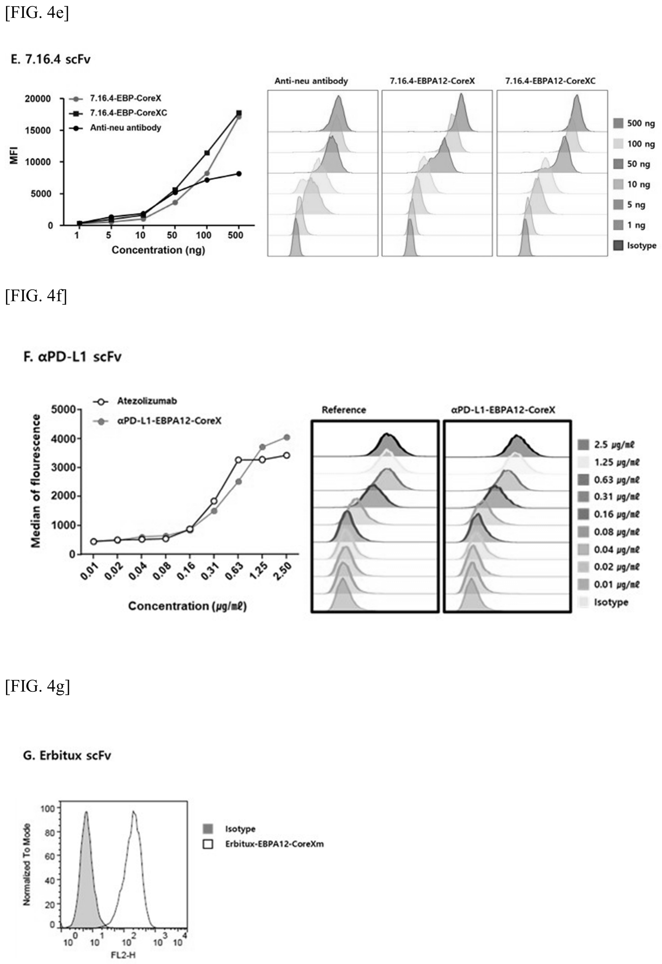

FIG. 4 shows results of identifying functionality of each fusion protein. FIG. 4A shows results of identifying a receptor recognition and cell inhibitory ability of an anti-Flt1 peptide fusion protein, and FIG. 4B shows a result of identifying a cell proliferation ability of a TPO stimulating peptide 14 (TSP14) fusion protein. FIG. 4C shows results of identifying an ability of an interferon-beta (IFN-β) cytokine fusion protein to increase intracellular receptor expression and regulate gene expression, and FIG. 4D shows results of identifying an enzymatic activity of adenosine deaminase2 (ADA2) enzyme fusion proteins and an antigen recognition ability according to the addition of antibody fragments. FIGS. 4E, 4F, and 4G show results of identifying an antigen recognition ability of 7.16.4, α-PD-L1, and Erbitux antibody fragment fusion proteins, respectively.

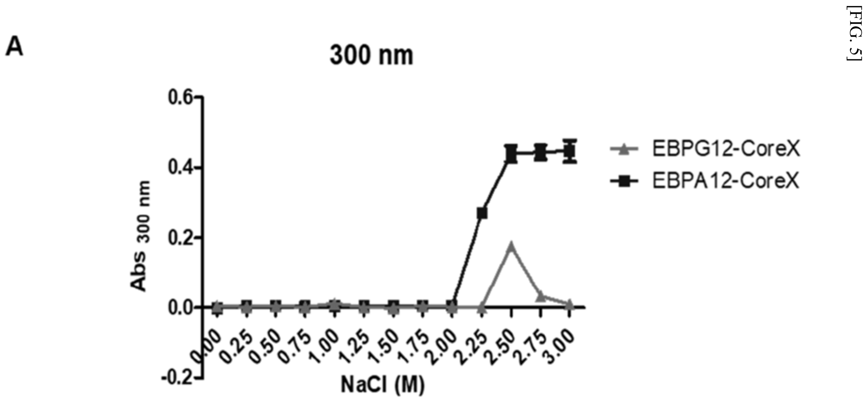

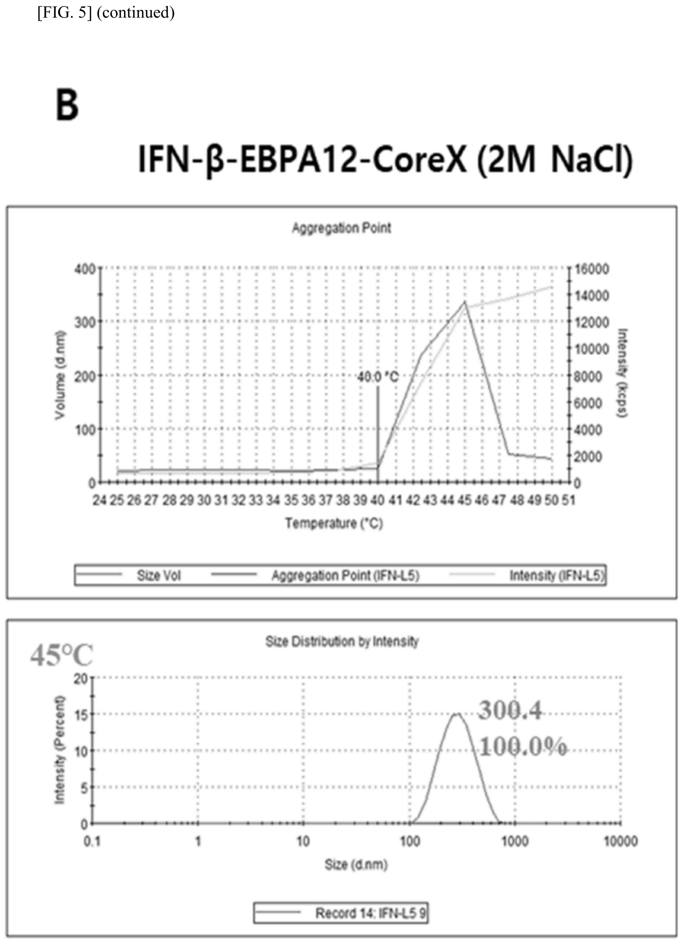

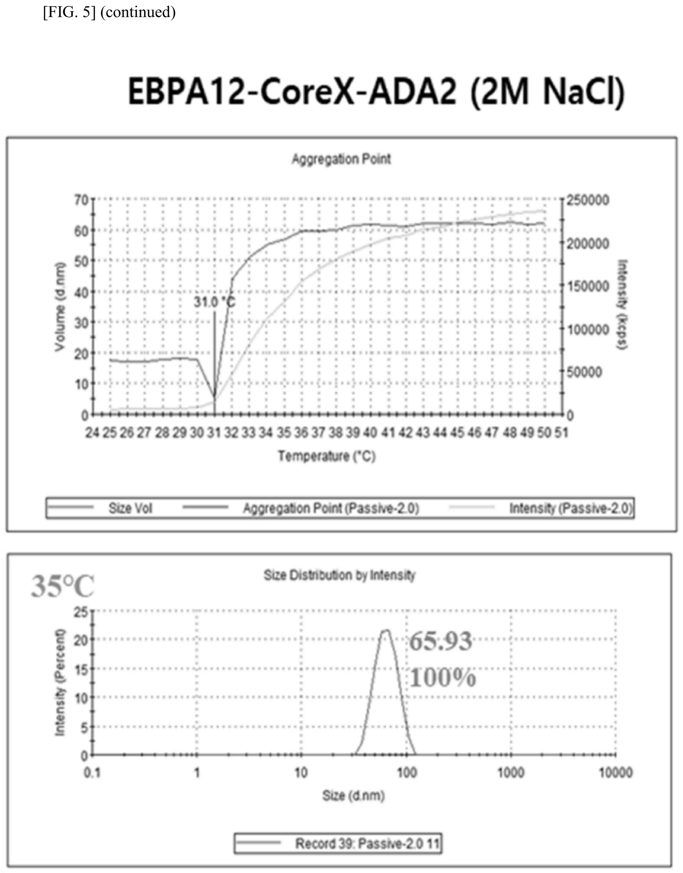

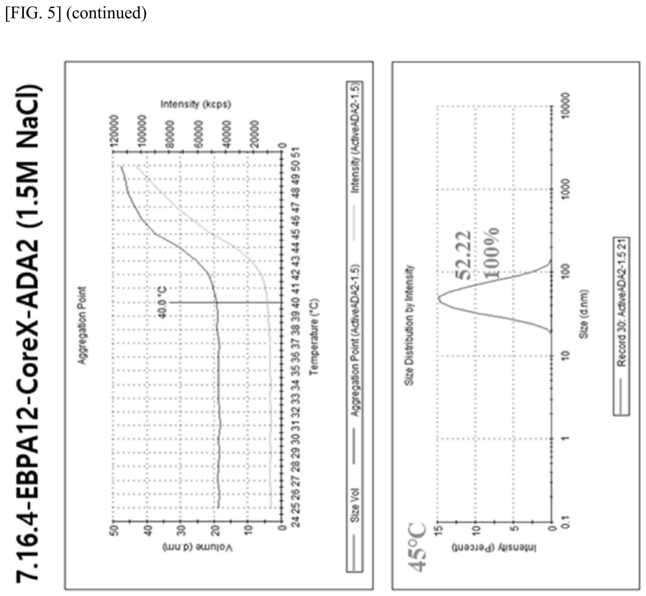

FIG. 5 shows results of identifying formation of a nanostructure of an EBPn-Fc protein, wherein FIG. 5A shows a result of measuring a change in turbidity according to salt concentration at room temperature, and FIG. 5B shows results of measuring a change in protein nanoparticle size according to salt concentration and temperature using a nano particle size analyzer (Zetasizer) which measures particle size by dynamic light scattering (DLS).

### Best Mode for Carrying Out the Invention

In the present disclosure, it was attempted to overcome shortcomings of ITC purification by developing an animal cell expression platform for an EBPn-Fc recombinant protein, in which hydrophilic EBP and a constant region (Fc) of an antibody are fused, thereby enabling high-purity purification with protein A or protein G. In addition, the present disclosure was completed as EBPn-Fc showed characteristics of forming nanostructures in response to salt concentration and temperature stimulation and was expressed as a recombinant protein in the form to which functional proteins such as peptides, proteins, and antibody fragments are fused to enable functional stabilization or targeting to antigens.

The present disclosure provides an animal cell expression cassette including a gene encoding a hydrophilic elastin-like polypeptide (EBPn), a gene encoding an antibody constant region (Fc), and a gene encoding a first protein for expression.

In addition, the animal cell expression cassette may further include a gene encoding a second protein for expression.

Preferably, the hydrophilic elastin-like polypeptide (EBPn) consists of [VPAXG VPAXG VPAXG VPAXG VPAXG VPAXG (SEQ ID NO: 1)]n or [VPGXG VPGXG VPGXG VPGXG VPGXG VPGXG (SEQ ID NO: 2)]n, where n may be an integer greater than or equal to 1 and the number of repetitions of the SEQ ID NO: 1 or SEQ ID NO: 2, and X may be selected from any amino acid except for proline, but is not limited thereto.

More preferably, the hydrophilic elastin-like polypeptide (EBPn) may be represented by any one amino acid sequence selected from SEQ ID NO: 3 to SEQ ID NO: 8, but is not limited thereto.

In the present disclosure, EBPn is a polypeptide in which EBP, a repeating unit of Val-Pro-(Ala or Gly)-Xaa-Gly pentapeptide, is repeated n times, and depending on the sequence of the repeating unit, one is EBPA whose sequence is Val-Pro-Ala-Xaa-Gly, and the other is EBPG whose sequence is Val-Pro-Gly-Xaa-Gly. The present inventors developed an animal cell expression platform technology for an EBPn-Fc recombinant protein by linking an antibody constant region (Fc) to the EBPn.

Preferably, the antibody constant region (Fc) may include a hinge region of IgG and a CH2-CH3 domain, and a cysteine residue in the hinge region of IgG may be additionally substituted with a serine residue, but are not limited thereto.

More preferably, the antibody constant region (Fc) may be represented by any one amino acid sequence selected from SEQ ID NO: 9 to SEQ ID NO: 12, but is not limited thereto.

Preferably, the protein for expression may be a peptide, a protein, or an antibody fragment, and more preferably, the protein for expression may be, but is not limited to, anti-Flt1 represented by an amino acid sequence of SEQ ID NO: 13, TSP14 represented by an amino acid sequence of SEQ ID NO: 14, IFN-β represented by an amino acid sequence of SEQ ID NO: 15, ADA2 represented by an amino acid sequence of SEQ ID NO: 16, 7.16.4 scFv represented by an amino acid sequence of SEQ ID NO: 17, α-PD-L1 scFv represented by an amino acid sequence of SEQ ID NO: 18, or Erbitux scFv represented by an amino acid sequence of SEQ ID NO: 19.

In addition, the present disclosure provides a recombinant expression vector including the animal cell expression cassette.

As used herein, the term "vector" refers to a self-replicating DNA molecule used to carry a clonal gene (or other pieces of clonal DNA).

As used herein, the term "expression vector" refers to a recombinant DNA molecule including a desired coding sequence and an appropriate nucleic acid sequence essential for expressing a coding sequence operably linked in a specific host organism. The expression vector may preferably include one or more selectable markers. The marker is a nucleic acid sequence having a characteristic that may be selected typically by chemical means and includes any gene capable of distinguishing transformed cells from non-transformed cells. Examples include, but are not limited to, antibiotic resistant genes such as ampicillin, kanamycin, geneticin (G418), bleomycin, hygromycin, and chloramphenicol, which may be appropriately selected by those skilled in the art.

Additionally, the present disclosure provides a cell line transfected with the recombinant expression vector.

In addition, the present disclosure provides a method of producing a protein for expression, including culturing a cell line and recovering a culture solution; and isolating and purifying a protein for expression fused with EBPn and Fc from the recovered culture solution.

Preferably, the purifying may be conducted via affinity chromatography using a protein A column, but is not limited thereto.

### Modes for Carrying Out the Invention

Hereinafter, the present disclosure will be described in more detail through examples. These examples are only intended to more specifically illustrate the present disclosure, and it will be apparent to those skilled in the art that the scope of the present disclosure is not limited by these examples according to the gist of the present disclosure.

### <Example 1> Production of an EBP-Fc fusion recombinant protein structure

For EBP, first cloning was performed after nucleotide synthesis, and for Fc, first cloning was performed similarly to EBP after PCR using a CH2-CH3 domain of the Fc portion of a mouse or human antibody as a template. The genetic sequences of each EBP and Fc were identified by sequencing (Bioneer). For the two types of EBPs having a pentapeptide repeating unit of Val-Pro-(Ala or Gly)-Xₐₐ-Gly[VP(A or G)XG], peptides were synthesized by constructing an amino acid sequence to make a ratio of Ala, Gly, and Ile at the fourth guest residue position (Xₐₐ) 1:4:1. After the first cloning, EBPn was produced by repeated fusion using restriction enzyme and T4 DNA ligase. For Fc, first cloning was performed after PCR using the CH2-CH3 domain of the Fc portion of mouse or human IgG antibodies as a template. At this time, after the EBPn and Fc were produced differently with one structure having two cysteine amino acid residues in the Fc hinge portion kept intact and the other structure changed to serine amino acids, the genetic base sequences of each were identified by sequencing (Bioneer).

For EBPn and Fc, second cloning was performed into the pSecTag2-Hygro B vector for animal cell expression using restriction enzyme and T4 DNA ligase to form EBPn-Fc. The base sequence of recombinant EBPn-Fc was identified by sequencing (Bioneer).

Afterwards, various protein genes such as peptides, proteins, and antibody fragments were used as a basic structure for fusion to the front or back of EBPn-Fc (FIG. 1). In FIG. 1A, each fusion protein was labeled as A, B, C, and D, and the structure of the EBPn-Fc fusion recombinant protein according to the expression order was simplified. The protein amino acid sequence of EBPn used in the basic structure is described in FIG. 1B.

For peptide fusion, anti-Flt1 was cloned in front of the EBPn-Fc basic structure after nucleotide synthesis, and TPO stimulating peptide (TSP) was cloned in front of the EBPn-Fc basic structure after synthesizing a 14 mer nucleotide (TSP14).

For protein fusion, IFN-β was cloned in two forms at the front and back of EBPn-Fc after PCR using cDNA of THP-1 cells as a template. ADA2 enzyme was produced in two forms, one obtained by performing PCR using cDNA of MCF7 cells as a template and then cloning to the front of EBPn-Fc, and the other obtained by adding the 7.16.4 antibody fragment to the front of EBPn-Fc and then cloning the ADA2 enzyme to the back to create a dual-function protein.

For antibody fragment fusion, the 7.16.4 antibody fragment portion was used as a template for PCR and then cloned to the front of EBPn-Fc. α-PD-L1 and Erbitux were also cloned in front of EBPn-Fc after PCR using the antibody fragment portion as a template.

### <Example 2> Labelling for EBPn-Fc fusion proteins

EBP is a VP(A or G)XG pentapeptide repeating unit, and VPAXG was labeled with EBPA and VPGXG with EBPG, respectively. The number n in EBP(A or G)n represents the number of repetitions of sequence number 1 [VPAXG VPAXG VPAXG VPAXG VPAXG VPAXG] or sequence number 2 [VPGXG VPGXG VPGXG VPGXG VPGXG VPGXG] (FIG. 1B). Fc was named CoreX and CoreX2 when the cysteine residue in the Fc hinge part of human IgG was removed, and CoreXm when the cysteine residue in the Fc hinge part of mouse IgG was removed. In addition, CoreXC represents Fc that retains the cysteine residues intact in the Fc hinge portion of human IgG. The name of the finally completed recombinant protein structure was used by sequentially connecting the names of EBPn and Fc, which were named differently depending on the name and type of the fusion protein. The amino acid sequences of each peptide, protein, and antibody fragment fused to the EBPn-Fc structure are shown in FIG. 1D.

### <Example 3> Comparison of ITC purification and affinity chromatography purification

The recombinant DNA constructed for expression of anti-Flt1-EBPA12-CoreXm was transfected into mammalian HEK293E cells to produce the EBPn-Fc fusion protein. For the produced cell culture solution, purification was separately carried out via ITC purification which is an existing EBP purification method and the affinity chromatography method using a protein A column. The purified protein was subjected to SDS-PAGE, and the gel was stained with EZ Gel staining solution (Daeil Lab Service) to determine the protein size and purity. Additionally, Western blot was performed for identification by targeting the Fc portion using anti-mouse IgG-HRP antibody (Jackson ImmunoResearch) (FIG. 2).

For ITC purification, the cell culture solution was mixed with high salt buffer (2.25 M glycine, 4.5 M NaCl, pH 8.9) at a ratio of 1:2 (culture solution:buffer) and reacted at 37°C for 45 minutes to aggregate the EBPn-Fc fusion protein. Then, after precipitation by centrifugation at room temperature for 10 minutes, suspension was performed in PBS followed by centrifugation at 4°C for 10 minutes to separate only the dissolved proteins. To increase the purity, the above process was repeated a total of three times (FIG. 2A).

In the affinity chromatography purification, the cell culture solution was mixed with 0.1 M sodium phosphate at pH 7.0 to meet a final concentration of 20 mM sodium phosphate, and then passed twice through a protein A (HiTrap rProtein A FF, GE) column to which a peristaltic Pump P-1 (GE) is connected. After washing the column with 20 mM sodium phosphate buffer, the EBPn-Fc fusion protein bound to the column was separated and purified with 0.1 M sodium citrate buffer at pH 4.0 (FIG. 2B).

In the attempt with the two purifications, it was found that the affinity chromatography purification using the antibody constant region (Fc) of the EBPn-Fc recombinant protein when expressed in animal cells had higher purification efficiency and purity than the ITC purification.

### <Example 4> Production of EBPn-Fc fusion proteins

Each recombinant DNA for expression was used to produce EBPn-Fc fusion proteins using the Expi293 expression system (Thermo), a transient expression system for animal cells. The expression of proteins in the produced cell culture solution was detected by performing SDS-PAGE and Western blot. SDS-PAGE gel was stained with EZ Gel staining solution (Daeil Lab Service) to detect protein expression, and Western blot was performed for detection by targeting the Fc portion with anti-mouse IgG-HRP antibody (Jackson ImmunoResearch) or anti-human IgG-HRP antibody (Jackson ImmunoResearch). In addition, the culture solution in which protein expression was identified was concentrated to less than 50 ml with Amicon Ultra-15, 30 k (Millipore), mixed with 0.1 M sodium phosphate at pH 7.0 to meet a final concentration of 20 mM sodium phosphate, and purified using a protein A (HiTrap rProtein A FF, GE) column connected with Peristaltic Pump P-1 (GE). The purified protein was subjected to buffer exchange using a PD-10 desalting column (GE), and its concentration was quantified using a BCA Protein Assay Kit (Thermo). The size and purity of the purified protein were verified using 0.5 to 1 µg of the quantified protein for SDS-PAGE and 0.05 to 0.1 µg for Western blot (FIG. 3). FIG. 3A shows results of identifying a protein purified by expressing EBP-Fc in its basic structure without a fusion protein, and FIGS. 3B and 3C show results of identifying a protein fused with the peptides Anti-Flt1 and TSP14, respectively. FIGS. 3D and 3E show results of identifying a protein fused with IFN-β cytokine and ADA2 enzyme, and FIGS. 3F, 3G, and 3H show results of identifying a protein fused with antibody fragments of 7.16.4, αPD-L1, and Erbitux, respectively.

### <Example 5> Identification of functionality of recombinant proteins

The experimental methods and results for identifying the functionality of each recombinant fusion protein are described in detail below for each protein.

For the function of the anti-FIt1 peptide fusion protein, the antiangiogenic effect was identified through inhibition of VEGF-induced HUVEC tube formation. HUVECs at 4 × 10⁴ per well were seeded onto 8-well chamber slides coated with Matrigel, stimulated with 50 ng/ml human VEGF 165, and then treated with various concentrations of anti-Flt1-EBPA12-CoreXm. The antiangiogenic effect was observed by randomly selecting three sites and dividing the length of the counted connecting cells by the total number of cells in the same field, using Avastin (bevacizumab), an inhibitor of the VEGF receptor, as a positive control. The results in the upper left of FIG. 4A show that tube formation of HUVECs induced by VEGF was significantly inhibited by 50% or greater at concentrations of 0.01 to 1 µg/ml of anti-Flt1-EBPA12-CoreXm. In addition, it is noted in the upper right bar graph of FIG. 4A that anti-Flt1-EBPA12-CoreX/Xm is able to bind to VEGFR, which is a VEGF receptor, to inhibit the binding with VEGF in a concentration-dependent manner by performing competitive ELISA. Competitive ELISA was performed by reacting human Flt1-Fc chimeric protein (0.5 µg/ml) with various concentrations of anti-Flt1-EBPA12-CoreX/Xm at room temperature for 2 hours, which was then added to a VEGF-coated 96-well plate to measure the amount of human Flt1-Fc chimeric protein that binds to VEGF. Compared with the control group, the anti-Flt1 peptide treated group (0.013, 0.13 ug/ml) did not inhibit the binding of VEGF and VEGFR1, but the anti-Flt1-EBPA12-CoreX treated group significantly inhibited the binding of VEGF and VEGFR1 by about 50% at concentrations of 0.001 to 1 ug/ml, with no inhibitory effect at a high concentration of 10 ug/ml. In the anti-Flt1-EBPA12-CoreXm treated group, the binding of VEGF and VEGFR1 was significantly inhibited at concentrations of 0.1 to 10 ug/ml. Anti-Flt1-EBPA12-CoreX and anti-Flt1-EBPA12-CoreXm showed similar efficacy, but the anti-Flt1-EBPA12-CoreXm was more effective when used at high concentrations.

The anti-angiogenic efficacy of the anti-Flt1 peptide fusion protein was identified by reduction in the CNV area in a laser-induced choroidal neovascularization (CNV) mouse model. Under anesthesia, choroidal neovascularization lesions were induced in the retina of C57BL/6 mice (n = 3-4) using a 532 nm wavelength diode laser, and 2 µl each of anti-Flt1 peptide and anti-Flt1-EBPA12-CoreX were injected once into the vitreous body of one eye on the day of laser injury. Eylea was used as a positive control. As a result, as noticed in the CNV lesion image and area graph below in FIG. 4A, the anti-Flt1 peptide treated group did not show a CNV area inhibitory effect, but intraocular administration of anti-Flt1-EBPA12-CoreX at various concentrations significantly inhibited the CNV area. In addition, it showed similar therapeutic effects to Eylea which is an existing treatment.

The activity of the TSP14 peptide fusion protein as a TPO receptor (TPOR) agonist was identified by increasing the proliferation of TPOR-overexpressing BaF3/MPL cells (FIG. 4B). BaF3/MPL cells at 5 × 10³ per well were seeded in 96-well plates and then cultured in the presence or absence of various concentrations of TPOR agonists for 48 hours, to measure the luminescent signal using a Cell Titer-Glo luminescent cell viability assay kit (Promega) in a Victor 3 1420 Multilabel Counter (Perkin Elmer). Human TPO synthetic protein (rhTPO) was used as a positive control. Both TSP14 and rhTPO stimulated the growth of BaF3/MPL cells in a concentration-dependent manner. As a result of calculating the relative cell proliferation ability based on 100 ng/ml rhTPO, which showed the maximum proliferation ability, the EC50 of TSP14 and rhTPO were 211.5 ng/ml and 1.7 ng/ml, respectively.

The immune activation effect of IFN-β1-EBPA12-CoreX was shown to increase the expression of MHC I. A549 cells were seeded in a 6-well plate at 0.3 × 10⁵ per well and treated with proteins for 48 hours. The cells were harvested and bound to human HLA antibodies, and the expression level of MHC I was measured using a flow cytometer. Human IFN-β1 synthetic protein was used as a positive control. In the left histogram graph of FIG. 4C, it is shown that the expression increased when IFN-β1-EBPA12-CoreX and human IFN-β1 synthetic protein were treated compared to the MHC I expression (green) in untreated A549 cells, and the expression levels by the two proteins were shown to be similar. The gene expression regulatory effect by IFN-β1-EBPA12-CoreXm was identified by the increase in TRAIL gene by recombinant protein treatment. A549 cells were seeded in a 6-well plate at 2 × 10⁵ per well and treated with protein for 24 and 48 hours, followed by RNA extraction. cDNA was synthesized from the extracted RNA, and denaturation was conducted using PCR premix (Bioneer) as primers for TRAIL gene (F: 5'-ACCAACGAGCTGAAGCAGAT-3', R: 5'-ACGGAGTTGCCACTTGACTT-3') and GAPDH gene (F: 5'-GGAGCGAGATCCCTCCAAAAT-3', R: 5'-GGCTGTTGTCATACTTCTCATGG-3') at 94°C for 5 minutes, followed by PCR reaction 20 times under temperature conditions of 94°C for 30 seconds, 57°C for 30 seconds, and 72°C for 60 seconds. PCR products were electrophoresed on a 1.5% agarose gel. The electrophoresis results on the right side of FIG. 4C showed that the expression of the TRAIL gene increased according to the treatment time of IFN-β1-EBPA12-CoreXm and human IFN-β1 synthetic protein, showing the greater increase by IFN-β1-EBPA12-CoreXm.

The enzymatic activity of the ADA2 enzyme fusion protein was identified by the degree of activity in decomposing adenosine into inosine. After reacting adenosine with the fusion protein, the amount of inosine formed by decomposition was quantified using the Adenosine Deaminase Activity Assay Kit (BioVisin, 328-100). As a result, it was found in the bar graph of FIG. 4D that the amount of inosine significantly increased when the ADA2 enzyme fusion protein was added. On the other hand, there was no significant difference depending on the presence of the 7.16.4 antibody fragment.

The antigen recognition ability of the ADA2 enzyme fusion protein fused with the 7.16.4 antibody fragment was detected by a concentration-dependent increase in binding to 4T1-neu cells in which neu antigen was overexpressed. After reacting with 5 × 10⁵ 4T1-neu cells at concentrations of 0.05 to 12.5 µg/ml for 30 minutes at 4°C, binding to cells was measured via a flow cytometer using anti-human IgG antibody (eBioscience). As a result, when the antigen recognition ability of ADA2 fused with the 7.16.4 antibody fragment was compared using an anti-neu antibody, it is noticed in the linear graph obtained from the fluorescence values in FIG. 4D that the degree of binding to the neu antigen was similar to that of the control anti-neu antibody.

The antigen recognition ability of the 7.16.4 antibody fragment fusion protein was identified by a concentration-dependent increase in binding to 4T1-neu cells in which the neu antigen is overexpressed (FIG. 4E). 3 × 10⁵ 4T1-neu cells were reacted with 1 to 500 ng of the fusion protein for 20 minutes at room temperature, and binding with the cells was measured by a flow cytometer using anti-human IgG antibody (eBioscience). Fluorescence values were calculated and graphed, and anti-neu antibody was used as a control. As a result, when the antigen recognition ability of anti-neu antibody and 7.16.4 antibody fragment fusion protein was compared, it was found that the degree of binding to the neu antigen was similar to that of the control anti-neu antibody at low concentrations, and the fluorescence value increased further in the 7.16.4 antibody fragment fusion protein at 50 ng or greater. Similar patterns were observed between CoreX and CoreXC, which differ in amino acid residues in Fc.

The antigen recognition ability of the α-PD-L1 antibody fragment fusion protein was identified by a concentration-dependent increase in binding to MDA-MB-231 cells, a breast cancer cell line (FIG. 4F). After reacting with 5 × 10⁵ MDA-MB-231 cells at concentrations of 0.01 to 2.5 µg/ml for 30 minutes at 4°C, binding to cells was measured by a flow cytometer using anti-human IgG antibody (eBioscience). The fluorescence values were calculated and graphed, and the comparison results between atezolizumab which is used clinically and the IC50 were 0.34 and 0.2, respectively, showing that the IC50 value of the a-PD-L1 antibody fragment fusion protein was about 0.1 lower.

The antigen recognition ability of the Erbitux antibody fragment fusion protein was identified by the binding to A549 cells, a lung cancer cell line (FIG. 4G). After reacting with 2 × 10⁵ A549 cells at room temperature for one hour, binding to cells was measured by a flow cytometer using anti-mouse IgG antibody (eBioscience). As a result, when the Erbitux antibody fragment fusion protein was treated to A549 cells, the fluorescence value increased compared to the control group, indicating that binding occurred by recognizing EGFR on A549 cells.

### <Example 6> Identification of nanostructure formation of recombinant proteins

The reactivity to the salt of the EBPn-Fc protein, a control of the fusion protein, was identified by the increase in turbidity with the increase in the salt concentration. After mixing the protein with salt at concentrations of 0.25 to 3.0 M, vortexing was performed at room temperature for 1 minute, and the absorbance was measured at a wavelength of 300 nm with nano-drops (FIG. 5A). As a result, EBPA12 showed a concentration-dependent increase in absorbance value at high salt concentrations of 2 M or higher, and EBPG12 showed an increase and then a decrease at 2.5 M. This shows that both types of EBPn-Fc are reactive to salt, but the reactivity differs depending on the type. Then, using the fusion protein, 10 µM protein was mixed with 1.5 to 2.0 M salt, the temperature was increased by 1.0°C at a time from 25 to 50°C, and the change in the particle size of the protein was measured by dynamic light scattering (DLS) using a nano particle size analyzer (Zetasizer). As a result, the change patterns in the temperature-dependent protein size varied depending on the type of fusion protein even though they had the same EBPn-Fc, and the protein size showed an increase and then became uniform when a certain temperature was reached at a high salt concentration. This demonstrates that the EBPn-Fc fusion protein is capable of forming nanostructures while maintaining the intrinsic properties of EBPn, which are responsive to salt and temperature (FIG. 5B).

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. An animal cell expression cassette comprising a gene encoding a hydrophilic elastin-like polypeptide (EBPn), a gene encoding an antibody constant region (Fc), and a gene encoding a first protein for expression.

2. The animal cell expression cassette of claim 1, wherein the animal cell expression cassette further comprises a gene encoding a second protein for expression.

3. The animal cell expression cassette of claim 1 or claim 2, wherein the hydrophilic elastin-like polypeptide (EBPn) consists of [VPAXG VPAXG VPAXG VPAXG VPAXG VPAXG (SEQ ID NO: 1)]n or [VPGXG VPGXG VPGXG VPGXG VPGXG VPGXG (SEQ ID NO: 2)]n, where n is an integer greater than or equal to 1 and the number of repetitions of the SEQ ID NO: 1 or SEQ ID NO: 2, and X is selected from any amino acid except for proline.

4. The animal cell expression cassette of claim 3, wherein the hydrophilic elastin-like polypeptide (EBPn) is represented by any one amino acid sequence selected from SEQ ID NO: 3 to SEQ ID NO: 8.

5. The animal cell expression cassette of claim 1 or claim 2, wherein the antibody constant region (Fc) comprises a hinge region of IgG and a CH2-CH3 domain.

6. The animal cell expression cassette of claim 5, wherein the antibody constant region (Fc) has a cysteine residue in the hinge region of IgG additionally substituted with a serine residue.

7. The animal cell expression cassette of claim 5, wherein the antibody constant region (Fc) is represented by any one amino acid sequence selected from SEQ ID NO: 9 to SEQ ID NO: 12.

8. The animal cell expression cassette of claim 1 or claim 2, wherein the protein for expression is a peptide, a protein, or an antibody fragment.

9. The animal cell expression cassette of claim 8, wherein the protein for expression is anti-Flt1 represented by an amino acid sequence of SEQ ID NO: 13, TSP14 represented by an amino acid sequence of SEQ ID NO: 14, IFN-β represented by an amino acid sequence of SEQ ID NO: 15, ADA2 represented by an amino acid sequence of SEQ ID NO: 16, 7.16.4 scFv represented by an amino acid sequence of SEQ ID NO: 17, α-PD-L1 scFv represented by an amino acid sequence of SEQ ID NO: 18, or Erbitux scFv represented by an amino acid sequence of SEQ ID NO: 19.

10. A recombinant expression vector comprising the animal cell expression cassette according to claim 1 or claim 2.

11. A cell line transfected with the recombinant expression vector according to claim 10.

12. A method of producing a protein for expression, the method comprising:
culturing the cell line according to claim 11 and recovering a culture solution; and
isolating and purifying a protein for expression fused with EBPn and Fc from the recovered culture solution.

13. The method of claim 12, wherein the purifying is conducted via affinity chromatography using a protein A column.
